# EUROPÄISCHE PATENTSCHRIFT

(11) **EP 2 478 863 B1**
(45) Veröffentlichungstag und Bekanntmachung des Hinweises auf die Patenterteilung: **02.10.2019**
(21) Anmeldenummer: 12000070.8
(22) Anmeldetag: 07.01.2012
(51) Int. Cl.: A61C 5/62, B05C 17/01, A46B 11/02

(54) **Applikator für eine Dentalflüssigkeit**
Applicator for a dental fluid
Applicateur pour un liquide dentaire

(30) Priorität: 21.01.2011 DE 102011009187
(43) Veröffentlichungstag der Anmeldung: 25.07.2012
(73) Patentinhaber: Kulzer GmbH, 63450 Hanau (DE)
(72) Erfinder: Vocke, Lutz, 61350 Bad Homburg (DE); Ziegler, Jan, 73312 Geislingen/Steige (DE)
(74) Vertreter: Bendele, Tanja

(56) Entgegenhaltungen:
- EP-A1- 1 188 455
- WO-A1-02/30314
- WO-A2-2008/057976
- DE-A1- 4 438 360
- JP-A- 2002 177 364

## Beschreibung

Die Erfindung betrifft einhändig bedienbare Applikatoren zum Aufbewahren und Applizieren einer Dentalflüssigkeit umfassend einen Behälter beinhaltend die Dentalflüssigkeit und eine Leitung mit der die Dentalflüssigkeit aus dem Behälter zu einer Oberfläche einer Applikatorspitze des Applikators leitbar ist.

Die Erfindung betrifft auch ein Verfahren zum Applizieren einer Dentalflüssigkeit mit einem solchen Applikator.

Applikatoren zum Auftragen einer Dentalflüssigkeit sind aus dem Stand der Technik bekannt. In der Zahnmedizin werden sie zum Auftragen von Dentalflüssigkeiten im Mundraum eines Patienten eingesetzt. Um auch schwer zugängliche Bereiche im Mundraum zugänglich zu machen, haben einige dieser Applikatoren, wie beispielsweise die aus der US 2003/0013066 A1 oder der US 2002/0090591 A1 bekannten Applikatoren eine abgewinkelte Applikatorspitze.

Aus der US 2003/0013066 A1 und der US 6,059,570 A ist die Verwendung eines Applikators mit einem frei beweglichen Förderkolben zum Austreiben des Inhalts bekannt.

Aus der US 2004/0197730 A1 ist ein Applikator bekannt, in dem das zu applizierende Fluid in einer abbrechbaren Ampulle am Griff des Applikators aufbewahrt wird. Die Ampulle wird abgebrochen und dadurch geöffnet. Die Applikatorspitze, die nach Art eines Pinsels aufgebaut ist, kann anschließend in die geöffnete Ampulle eingetaucht werden, um die Applikatorspitze mit der Dentalflüssigkeit zu benetzen. Die benetzte Applikatorspitze kann dann anschließend zum Auftragen der Dentalflüssigkeit verwendet werden.

Nachteilig ist hieran, dass der Applikator mit beiden Händen bedient werden muss und auch bei mehrmaligem Eintauchen aufwendig in der Bedienung ist.

Ein einhändig bedienbarer Applikator ist aus der US 2009/0060624 A1 bekannt. Dieser ist mit einem aufwendig konstruierten Vortriebsmechanismus ausgestattet, der einen Verdrängerkolben vortreibt, mit dem die Dentalflüssigkeit aus dem Applikator gedrückt wird. Die Applikatorspitze und der Vortriebsmechanismus sind mit einer Kappe abgedeckt, um ein ungewolltes Austreten der Dentalflüssigkeit aus dem Applikator zu verhindern. In den Dentalflüssigkeiten können chemisch aggressive Substanzen enthalten sein, die nicht ungewollt aus dem Applikator entweichen sollen.

Ein solcher Applikator hat den Nachteil, dass trotz der einfacheren Anwendung immer noch die Kappen gelöst werden müssen, um den Applikator einsatzbereit zu machen. Zudem können sich die Kappen ungewollt lösen und Dentalflüssigkeit kann sich in der Kappe über der Applikatorspitze sammeln, die dann beim Öffnen der Kappe austritt. Ferner kann der Behälter des Applikators aufgrund der chemischen Aggressivität der Dentalflüssigkeit undicht werden.

EP 1 188 455 A1 offenbart eine Vorrichtung zum Austragen des Inhalts eines abgeschlossenen flüssigkeitsgefüllten Behältnisses. Nachteilig an dem Behältnis der D1 ist, dass sich die Flüssigkeit im Kolben befindet und über die innenliegende, hohle Spitze über einen Lueranschluss in einen langes Rohr 15 befördert wird und durch die innenliegende hohle Spitze nicht restentleert werden kann. WO02/30314 A1 offenbart eine Spritze mit einem Mehrkammersystem und einer Folie, die vor der Applikation durchstoßen wird.

Die Aufgabe der Erfindung besteht also darin, die Nachteile des Stands der Technik zu überwinden. Insbesondere soll eine möglichst einfache und unkomplizierte Bedienung des Applikators ermöglicht werden, die die Abläufe während der Behandlung möglichst wenig beeinträchtigt. Zudem soll eine einfache Handhabbarkeit durch einen möglichst unkomplizierten und einfachen Aufbau erreicht werden. Gleichzeitig soll der Applikator auch ein möglichst sicheres Behältnis für die Dentalflüssigkeit bieten, damit die Umgebung nicht kontaminiert wird.

Die Aufgabe der Erfindung wird durch eine Vorrichtung gemäß Anspruch 1 gelöst.

Unter einer Dentalflüssigkeit wird vorliegend eine Flüssigkeit, Suspension oder ein Gemisch umfassend eine Flüssigkeit verstanden, die beziehungsweise das ausreichend dünnflüssig ist, um ein Applizieren der Dentalflüssigkeit zu ermöglichen. Der Druck, der mit einer Hand nach Art einer Spritze aufgebracht wird, kann dabei ausreichend sein, um die Dentalflüssigkeit durch die Leitung an die Oberfläche der Applikatorspitze und gegebenenfalls durch einen Verteiler zu drücken. Typische Dentalflüssigkeiten sind beispielsweise Bleichmittel, Adhäsive, Füllmaterial, Desinfektionsmittel und Beschichtungen.

Bis zum Öffnen des Behälters im Applikator dichtet die Folie den Behälter ab und verhindert ein Austreten der
Dentalflüssigkeit und damit eine Kontamination der Umgebung sowie einen Verlust der Dentalflüssigkeit. Die Folie kann erfindungsgemäß eine beachtliche Dicke aufweisen, solange sie noch mechanisch durch einen manuellen Druck zu durchstoßen ist.

Es kann vorgesehen sein, dass die Folie eine Aluminium-Mehrfachverbund-Folie ist. Die Folie kann durch ein thermisches Siegelverfahren oder Schweißverfahren auf eine offenseitige Behälterkante aufgeschweißt werden.

Die Beschichtung bewirkt ebenfalls eine Abdichtung des Behälters. Mit der Beschichtung wird eine zusätzliche Barriere für die Dentalflüssigkeit geschaffen und damit ebenfalls eine Kontamination der Umgebung mit der Dentalflüssigkeit oder Bestandteilen davon verhindert. Die Beschichtung kann dazu auf die Inhaltsstoffe der Dentalflüssigkeit abgestimmt sein.

Es kann auch vorgesehen sein, dass der Applikator zumindest einen weiteren Behälter mit zumindest einer weiteren Komponente einer Dentalflüssigkeit umfasst, wobei eine Mischung der beiden Behälterinhalte eine gemischte Dentalflüssigkeit ergibt. Dies kann vorteilhaft sein, wenn die beiden Komponenten der gemischten Dentalflüssigkeit miteinander reagieren und daher erst kurz vor der Applikation beziehungsweise Anwendung beim Patienten gemischt werden sollen.

Es ist vorgesehen, dass ein Dorn im Applikator angeordnet ist, mit dem die Folie durchstechbar ist, wobei der Behälter mit durchstochener Folie geöffnet ist und wobei der Dorn vorzugsweise im Bereich der Leitung und des Behälters angeordnet ist, besonders bevorzugt als Hohldorn ausgebildet ist, der ein erstes Ende der Leitung bildet. Statt einem Dorn kann auch eine Klinge vorgesehen sein, mit der die Folie aufgeschnitten wird.

Dies hat den Vorteil, dass die Folie des Behälters auf den Dorn gedrückt werden kann, um den Behälter zu öffnen und damit den Applikator einsatzbereit zu machen. Eine zusätzliche Kappe zum Abdecken der Applikatorspitze kann so gespart werden. Der Behälter ist zum Dorn so gelagert, dass der Vortriebsmechanismus, der zum Austreiben der Dentalflüssigkeit aus dem Applikator verwendet wird, auch dazu verwendet wird, die Folie mit dem Dorn zu durchstoßen oder aufzuschlitzen. Dann wird die Öffnung des Behälters mit derselben Kraft geöffnet, mit der auch die Dentalflüssigkeit ausgetragen wird. Dadurch wird eine einhändige Bedienbarkeit des Applikators nicht nur beim Austragen der Dentalflüssigkeit erreicht, sondern auch beim Öffnen des Behälters, beziehungsweise beim Einsatzbereitmachen des Applikators.

Ferner kann bei beschichteten Behältern vorgesehen sein, dass die Beschichtung eine hydrophobe und/oder chemisch resistente Beschichtung ist, die vorzugsweise bis über 200° C temperaturstabil ist.

Auch kann bei beschichteten Behältern vorgesehen sein, dass die Beschichtung eine porenfreie Polymerbeschichtung, vorzugsweise eine Parylene-Beschichtung ist.

Eine solche porenfreie Polymerbeschichtung ist für Dentalflüssigkeiten aufgrund ihrer chemischen Beständigkeit besonders geeignet. Die Porenfreiheit verhindert dabei ein Durchsickern der Dentalflüssigkeit oder Bestandteilen davon bis zur eigentlichen Behälterwand. Für die Beschichtung kann ein Material verwendet werden, das für die Behälterwandung keine ausreichende mechanische Stabilität aufweist oder zu kostenaufwendig wäre. Poly(para-Xylylen), kurz Parylene, kann beispielsweise durch CVD (chemische Gasphasenabscheidung) als Schicht mit Schichtdicken zwischen einigen 100 nm bis einigen 10 µm aufgetragen werden. Die Parylene-Beschichtung ist insbesondere für Dentalflüssigkeiten aufgrund der chemischen Beständigkeit besonders gut geeignet. Zudem ist die thermische Beständigkeit und mechanische Stabilität von Parylene bei der Herstellung und der Verwendung des Applikators vorteilhaft. Auch die Abriebfestigkeit der Parylene-Beschichtung und die Möglichkeit Parylene-Beschichtungen bei niedrigen Temperaturen aufzutragen, ist dabei von besonderem Vorteil.

Da die für Dentalflüssigkeiten gerne verwendeten Lösungsmittel leicht flüchtig sind, ist es sinnvoll, eine produktangepasste Barrierewirkung bereitzustellen. Da die im Markt erhältlichen Massenkunststoffe in Ihrer Reinform keine ausreichende Barrierewirkung erzielen, muss der Behälter über zusätzliche Veredelungsverfahren mit einem weiteren Leistungsmerkmal ausgestattet werden. Die Barrierewirkung durch die sogenannten Parylenebeschichtung ist gegenüber solchen Lösungsmitteln verbessert. Dies wurde experimentell durch Lagertests und Überprüfung von Masseverlusten eines Prüfmediums beobachtet.

Es kann vorgesehen sein, dass die Dicke der Beschichtung 0,1 µm bis 100 µm ist, vorzugsweise zwischen 1 µm und 10 µm.

Erfindungsgemäß kann auch vorgesehen sein, dass die Leitung flexibel ist und einen Kanal zum Durchleiten der Dentalflüssigkeit aus dem Behälter umfasst, wobei der Kanal auf einer Seite mit dem Behälter durch eine Fluidverbindung verbunden ist.

Durch die Flexibilität der Leitung kann der Applikator auf die Zugänglichkeit der zu behandelnden Stelle im Mundraum angepasst werden.

Ein während einer Behandlungssituation besonders gut einzusetzender Applikator ergibt sich, wenn vorgesehen ist, dass der Applikator einen Verteiler zum Verteilen der Dentalflüssigkeit umfasst, insbesondere an einem zweiten Ende der Leitung an der Applikatorspitze, wobei der Verteiler die Oberfläche der Applikatorspitze bildet.

Dabei kann vorgesehen sein, dass der Verteiler ein offenporiges Material umfasst oder aus einem offenporigen Material besteht.

Ferner kann vorgesehen sein, dass das offenporige Material ein Schwamm und/oder Schaumstoff ist, vorzugsweise der Verteiler ein Schaumstoffstanzling ist.

Auch kann vorgesehen sein, dass das offenporige Material eine Porengröße von 50 nm bis 500 µm, bevorzugt von 0,5 µm bis 100 µm, besonders bevorzugt von 5 µm bis 50 µm hat.

Des Weiteren kann vorgesehen sein, dass der Verteiler austauschbar und/oder auswechselbar ist, vorzugsweise auf das erste Ende der Leitung aufsteckbar ist. Durch diese Maßnahme ergibt sich ein variabel einsetzbarer Applikator.

Ferner kann vorgesehen sein, dass der Verteiler eine Vielzahl von Kanälen umfasst, die mit dem Kanal der Leitung über Fluidverbindungen verbunden sind.

Eine weitere Ausgestaltung der Erfindung sieht vor, dass die flexible Leitung um mindestens 45° biegbar ist, vorzugsweise um mindestens 90° biegbar ist, besonders bevorzugt um mindestens 100°.

Eine Weiterbildung der Erfindung sieht vor, dass die flexible Leitung plastisch deformierbar ist. Besonders bevorzugte Ausführungen der Erfindung können vorsehen, dass die Dentalflüssigkeit Wasser, Aceton, Ethanol, Isopropanol, Ethylessigester, MMA und/oder tert.-Butanon beinhaltet.

Eine besonders dichte Ausführungsform eines erfindungsgemäßen Applikators kann dadurch erreicht werden, wenn vorgesehen ist, dass der Behälter doppelwandig ist, wobei vorzugsweise die innere Wand zumindest bereichsweise aus einem hochdichten Kunststoff gefertigt ist und/oder die äußere Wand aus einem Cyclo-Olefin-Copolymer (COC) gefertigt ist.

Es ist erfindungsgemäß vorgesehen, dass der Applikator einen Verdrängerkolben zum Austreiben der Dentalflüssigkeit aus dem Behälter umfasst.

Dabei kann vorgesehen sein, dass der Applikator eine Rastung umfasst, mit der der Verdrängerkolben gegen den Behälter arretierbar ist.

Ferner kann vorgesehen sein, dass die Rastung eine Vielzahl von Raststufen hat, wobei die Raststufen manuell überwindbar sind, so dass die Dentalflüssigkeit portionsweise aus dem Behälter applizierbar ist.

Durch die Rastung wird eine haptische und fühlbare Portionierung der Dentalflüssigkeit bei einer manuellen Bedienung des Applikators ermöglicht. Auch kann anhand der Raste, in der sich der Verdrängerkolben befindet, abgelesen werden, wie viel der Dentalflüssigkeit noch in dem Applikator enthalten ist.

Ferner kann vorgesehen sein, dass der Applikator einen Volumenverdränger zur Restentleerung des Behälters umfasst.

Der Volumenverdränger kann an dem Verdrängerkolben angeordnet sein. Er dient dazu, auch den letzten Rest der Dentalflüssigkeit aus dem Applikator zu pressen und damit das gesamte Innenvolumen des Behälters nutzbar zu machen. Dazu kann der Volumenverdränger an die innere Form des Behälters im Bereich der Leitung und auch der Leitung beziehungsweise dem Kanal angepasst sein.

Ferner kann die Leitung, beziehungsweise der Kanal der Leitung auch zumindest bereichsweise mit dem Fluid gefüllt sein, so dass der Kanal bzw. die Leitung auch Teil des Behälters ist. Der Kanal der Leitung muss also nicht leer sein.

Im Nachfolgenden wird ein Verfahren zum Applizieren einer Dentalflüssigkeit mit einem Applikator gemäß Anspruch 1 beschrieben, bei dem die Folie und/oder der Behälter geöffnet wird, insbesondere durchstochen wird, bevor die Dentalflüssigkeit appliziert wird. Dabei kann vorgesehen sein, dass die Applikatorspitze, insbesondere ein Verteiler des Applikators, über die mit der Dentalflüssigkeit zu beschichtende Oberfläche gestrichen wird und gleichzeitig ein Druck auf die Dentalflüssigkeit im Inneren des Behälters ausgeübt wird, so dass die Dentalflüssigkeit über die flexible Leitung und die Applikatorspitze, insbesondere den Verteiler appliziert wird.

Dabei ist wiederum vorgesehen, dass der Druck auf die Dentalflüssigkeit über einen Verdrängerkolben ausgeübt wird.

Eine Weiterbildung des Verfahrens kann vorsehen, dass die Verdrängung der Dentalflüssigkeit durch den Druck zumindest zu einer bereichsweisen Benetzung der Oberfläche der Applikatorspitze, insbesondere des Verteilers führt.

Auch kann vorgesehen sein, dass der Füllstand des Behälters durch Verwendung einer Rasterung haptisch und/oder akustisch bestimmt wird.

Besonders vorteilhaft ist, dass die Dentalflüssigkeit einhändig appliziert wird.

Schließlich kann vorgesehen sein, dass ein für die zu benetzende Oberfläche geeigneter Verteiler an der Applikatorspitze angebracht wird, bevor die Dentalflüssigkeit auf die zu benetzende Oberfläche appliziert wird.

Der Erfindung liegt die überraschende Erkenntnis zugrunde, dass es durch die Beschichtung und durch die Folie auch jeweils einzeln schon gelingt, die Dichtigkeit des Applikators zu verbessern, wobei es gleichzeitig gelingt, mit dem selben Handgriff, der zum Anwenden des Applikators benötigt wird, den Applikator einsatzbereit zu machen. Die Beschichtung stellt eine einfache Möglichkeit dar, den Behälter für die Dentalflüssigkeit besser und nachhaltiger abzudichten, so dass die Dentalflüssigkeit nicht so leicht aus dem Behälter austreten kann. Die Folie, die ebenfalls zum Abdichten des Behälters dient, ermöglicht dabei ein einfaches Öffnen des Behälters, indem sie mechanisch geöffnet wird. Dazu reicht es aus, die Folie aufzuschlitzen oder zu durchbohren. Ebenso könnte die Folie gestanzt werden. Dazu kann die selbe Kraft verwendet werden, die zum Austreiben der Dentalflüssigkeit aufgebracht wird. So kann beispielsweise der Behälter durch Ausüben eines Drucks auf einen Verdrängerkolben auf einen Dorn gepresst werden, bis die Folie nachgibt.

Besonders die Kombination von Folie und Beschichtung ermöglicht es, einen geeigneten Behälter für einen Applikator aufzubauen, der die mechanische Stabilität eines Materials für die Behälterwand und/oder die Folie und gleichzeitig die chemische Beständigkeit eines zweiten Materials der Beschichtung gegenüber der Dentalflüssigkeit nutzt. Die Kombination ermöglicht also besonders dünne Behälterwände beziehungsweise Folien aufzubauen.

Die vorliegende Erfindung liefert also einen mehrteiligen Kunststoffbehälter zum direkten Auftragen dentaler Flüssigkeiten auf Oberflächen im Bereich der Mundhöhle, beziehungsweise ein Verpackungssystem zur sogenannten Einmalanwendung (auch "single dosage" oder "single usage" genannt). Es ermöglicht dem Anwender eine schnelle, einfach handhabbare und sichere Versorgung von Patienten.

Die zu applizierende Dentalflüssigkeit befindet sich in dem mit einer Folie verschlossenen Behälter. Durch einen Druck auf den Behälter, insbesondere die Behälterrückseite, wird das Verpackungssystem zur Anwendung aktiviert. Hierzu wird die Folie durch ein dornenartiges Gegenstück an der Innenseite der Applikatorspitze oder der Innenseite des Applikators durchstoßen.

Eine für die dentale Anwendung relevante Aceton-haltige Flüssigkeit ("Dentalbonding") wird separiert in dem vorgesehenen Behälter vorbereitet. Dadurch ist die Dentalflüssigkeit räumlich von der Applikatorspitze getrennt. Beide Teile sind auch im inaktiven Zustand (Auslieferungszustand) ineinander gesteckt.

Nach der Aktivierung wird die bevorratete Dentalflüssigkeit durch einen innenliegend konstruierten Kanal zur zu benetzenden Applikationsspitze transportiert. Der Aktivierungsvorgang wird durch einen speziell geformten Kunststoffzylinder im Inneren des Applikators ausgelöst. Die Anordnung und Formgestalt des Zylinders beinhaltet einen Dorn oder eine Klinge, mit dessen oder deren Hilfe die Folie durchstoßen oder aufgeschnitten wird. Zum Zweck eines optimalen Öffnungsverhaltens sind der Dorn beziehungsweise die Klinge und die Innenseite des Behälters maßlich aufeinander abgestimmt. Der Flüssigkeitstransport aus dem Behälter in Richtung Applikationsspitze erfolgt durch Volumenverdrängung des Behälter-Innenraums.

Durch Verbindung von Applikator und Bevorratungsbehälter wird eine einhändige Applikation möglich. Die Verdrängung des Innenvolumens führt zu einer Benetzung der Applikatorspitze. Eine Doppelkammer kann zur Optimierung der Barrierewirkung des Kunststoffs für das empfindliche und aggressive Aceton-Gemisch vorgesehen sein.

Erfindungsgemäße Applikatoren ermöglichen die einhändige Aktivierung, eine zeitsparende Anwendung und eine Reduzierung der Vorbereitungszeit.

Im Folgenden werden Ausführungsbeispiele der Erfindung anhand von drei schematisch dargestellten Figuren erläutert, ohne jedoch dabei die Erfindung zu beschränken. Dabei zeigt:
Figur 1: eine schematische Querschnittansicht eines nicht erfindungsgemäßen Applikators;
Figur 2: eine schematische Querschnittansicht eines erfindungsgemäßen Applikators; und
Figur 3: eine schematische Seitenansicht des Applikators nach Figur 2.

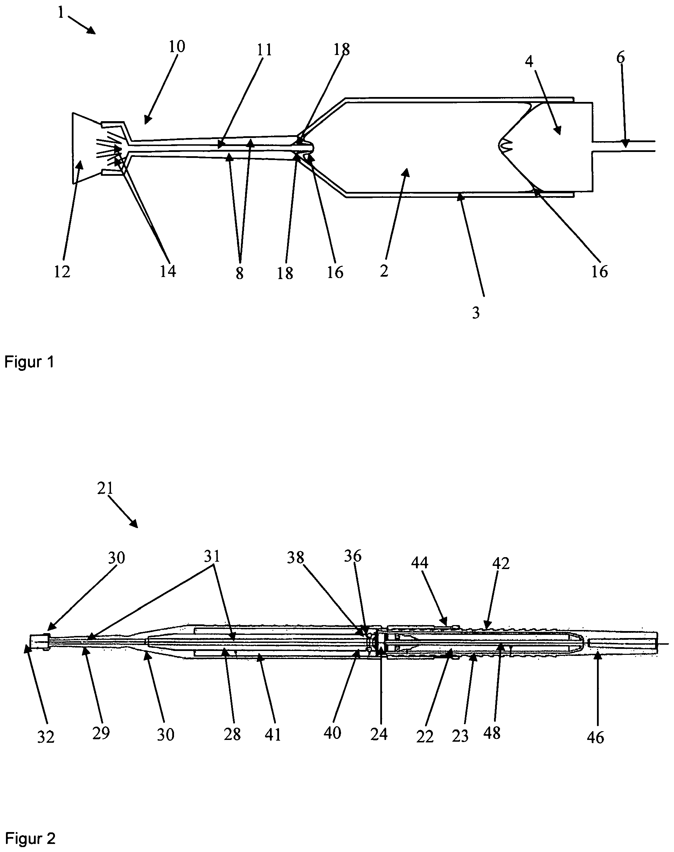

Figur 1 zeigt eine schematische Querschnittansicht eines nicht erfindungsgemäßen Applikators 1, der eine Dentalflüssigkeit 2 beinhaltet. Die Dentalflüssigkeit 2 befindet sich in einem Behälter 3 mit zylindrischem Innenraum. An einem Ende des Applikators 1 ist ein zylindrischer Verdrängerkolben 4 in dem Behälter 3 angeordnet. Der Verdrängerkolben 4 ist entlang der Zylinderachse beweglich im Behälter 3 angeordnet und kann über eine Stange 6, die fest am Verdrängerkolben 4 angebracht ist, vorgetrieben werden.

Auf der Vorderseite des Applikators 1 ist eine flexible Leitung 8 angeordnet, die einen Kanal 11 umfasst, der das Innere des Behälters 3 mit einer Applikatorspitze 10 verbindet. Die Form des Verdrängerkolbens 4 ist frontseitig an die Form des Behälters 3 im Bereich der Einmündung in die Leitung 8 angepasst. Diese Ausformung stellt einen Volumenverdränger dar, der dafür sorgt, dass die gesamte Dentalflüssigkeit 2 mit dem Verdrängerkolben 4 aus dem Inneren des Behälters 3 ausgetrieben werden kann.

Der Kanal 11 der Leitung 8 mündet im Bereich der Applikatorspitze 10 in einen Verteiler 12, der aus einem porösen, offenporigen Schaumstoff aufgebaut ist. im Verteiler 12 sind zusätzlich Kanäle 14 vorgesehen, die das Verteilen der Dentalflüssigkeit 2 auf der Oberfläche des Verteilers 12 bei einer Anwendung des Applikators 1 unterstützen sollen. Der Verteiler 12 kann manuell in die Applikatorspitze 10 eingesetzt und ausgetauscht werden.

Die Dentalflüssigkeit 2 ist durch eine flexible Folie 16 im Applikator 1 eingeschlossen. Die Folie 16 bildet die Innenwand des Behälters 3. Im Bereich des Eintritts des Kanals 11 der Leitung 8 in den Innenraum des Behälters 3 ist ein Hohldorn 18 angeordnet, mit dem die Folie 16 aufgeschlitzt werden kann. Wenn ein Druck auf den Verdrängerkolben 4 ausgeübt wird, wird die Dentalflüssigkeit 2 mit der Folie 16 auf den Hohldorn 18 gedrückt und dadurch die Folie 16 geöffnet. Bei einem weiteren Druck auf den Verdrängerkolben 4 wird die Dentalflüssigkeit 2 aus dem Behälter 3 durch den Kanal 11 in den Verteiler 12 gedrückt. Dort tränkt die Dentalflüssigkeit 2 den Verteiler 12 und tritt schließlich an die Oberfläche des Verteilers 12 aus. Die mit der Dentalflüssigkeit 2 benetzte Oberfläche des Verteilers 12 wird dann über die zu benetzende Oberfläche im Mundraum eines Patienten gestrichen. Die Dentalflüssigkeit 2 überträgt sich auf die zu behandelnde Oberfläche. Ein ungleichmäßiges Drücken auf den Verdrängerkolben 4 wird durch die Aufnahmefähigkeit des porösen Verteilers 12 ausgeglichen.

Figur 2 zeigt eine schematische Querschnittansicht eines erfindungsgemäßen Applikators 21. Bei dieser Ausführungsform ist eine Dentalflüssigkeit 22 in einem beschichteten Behälter 23 enthalten. An einem Ende des Behälters 23 ist im Behälter 23 ein Verdrängerkolben 24 angeordnet, der gegen den Behälter 23 verschiebbar ist.

Der Behälter 23 ist auch gegen eine Leitung 28 verschiebbar. Der Verdrängerkolben 24 kann also mit der Leitung 28 verbunden sein oder die Leitung 28 ist derart aufgebaut, dass sich die Wände des Behälters 23 zwar gegen die Leitung 28 bewegen lassen, der Verdrängerkolben 24 jedoch nicht. Die gesamte Leitung 28, 29 umfasst eine flexible Leitung 29 im Bereich einer Applikatorspitze 30. In den beiden Teilen der Leitung 28, 29 ist ein Kanal 31 angeordnet, der das Innere des Behälters 23 mit der Applikatorspitze 30 beziehungsweise einem daran angeordneten Verteiler 32 verbindet. Der Verteiler 32 ist ein Schaumstoffstanzling, der in eine Öffnung am äußeren Ende der flexiblen Leitung 29 eingesetzt ist.

An dem Ende des Behälters 23, das der Leitung 28 zugewandt ist, ist der Behälter durch eine Folie 36 abgeschlossen. Die Folie 36 liegt auf Dorne 38 auf, die dazu geeignet sind, die Folie 36 und damit den Behälter 23 zu öffnen. Am Eintritt der Leitung 28 in den Behälter 23 ist eine Dichtung 40 angeordnet, die verhindert, dass Dentalflüssigkeit 22 in einen Hohlraum 41 eindringt, der die Seitenwände des Behälters 23 aufnimmt, wenn dieser über die Leitung 28 geschoben wird. Beim Einschieben der Seitenwände des Behälters 23 in den Hohlraum 41 bleibt der Verdrängerkolben 24 relativ zur Leitung 28 an der gleichen Stelle, so dass die Dentalflüssigkeit 22 aus dem Inneren des Behälters 23 heraus durch die Leitung 28, 29 in den Verteiler 32 gedrückt wird. Durch den offenporigen Verteiler 32 dringt die Dentalflüssigkeit 22 bis zur Oberfläche des Verteilers 32 vor. Dort kann sie an die zu behandelnde Stelle abgegeben, beziehungsweise auf die zu behandelnde Stelle aufgetragen werden.

Die Innenflächen des Behälters 23 und vorzugsweise auch die dem Inneren des Behälters 23 zugewandte Fläche der Folie 36 sind mit Parylene beschichtet, um eine Auflösung der Behälterwände und der Folie 36 durch die chemisch aggressive Dentalflüssigkeit 22 zu verhindern. Die Dentalflüssigkeit 22 kann beispielsweise Aceton, Ethylessigester, MMA und/oder tert.-Butanon enthalten. Diese chemisch aggressiven Substanzen können die Polymere, aus denen der Behälter 23 und andere Teile des Applikators 21 aufgebaut sind, auflösen. Dadurch könnte der Behälter 23 porös werden und Dentalflüssigkeit 22 nach außen dringen. Dies wird durch die Parylene-Beschichtung verhindert. Die Folie 36 kann eine dünne unbeschichtete Metallfolie sein, die gegen die in der Dentalflüssigkeit enthaltenen Substanzen resistent ist.

Parylene-Beschichtungen sind extrem dünne, porenfreie Polymer-Beschichtungen, die zu vielerlei Zwecken verwendet werden. Parylene ist ein inertes, hydrophobes, optisch transparentes, biokompatibles, polymeres Beschichtungsmaterial mit einem weiten industriellen Anwendungsspektrum.

Die Parylene-Beschichtung wird im Vakuum durch Kondensationen aus der Gasphase als porenfreier und transparenter Polymerfilm auf das Substratmaterial (Behälterwandungen, innen und außen) aufgetragen. Aufgrund der gasförmigen Abscheidung erreicht und beschichtet Parylene auch Bereiche und Strukturen welche mit flüssigkeitsbasierten Verfahren nicht beschichtbar sind, wie zum Beispiel scharfe Ränder und Spitzen oder enge und tiefe Spalten. In einem Arbeitsgang können Beschichtungsdicken von 0,2 bis 100 µm aufgebracht werden.

An der Außenfläche des Behälters 23 ist eine Rastung 42 angeordnet, die in eine Gegenrastung 44 greift, die fest mit der Leitung 28 verbunden ist. An dem der Austragsspitze 30 gegenüberliegenden Ende des Applikators 21 ist ein Griff 46 angeordnet, über den der Behälter 23 in den Hohlraum 41 gedrückt werden kann. Im Inneren des Behälters 23 ist ein Volumenverdränger 48 angeordnet, der sich in die Leitung 28 schiebt. Der Volumenverdränger 48 ist eine zylindrische Röhre, deren Außendurchmesser etwas größer ist als der Innendurchmesser der Leitung 28. Durch die Differenz der Durchmesser ist sichergestellt, dass die Dentalflüssigkeit 22 auch bei eingeschobenem Volumenverdränger 48 noch durch die Leitung 28 gedrückt wird. Durch den eingeschobenen Volumenverdränger 48 wird der effektive Querschnitt der Leitung 28 verringert. Dadurch wird das Totvolumen des Applikators 21 reduziert. Es verbleibt also weniger der Dentalflüssigkeit 22 ungenutzt im Applikator 21.

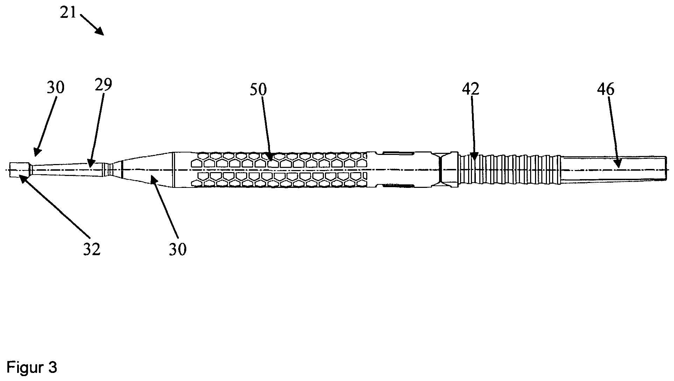

Figur 3 zeigt eine schematische Seitenansicht des Applikators 21 nach Figur 2. An der flexiblen Leitung 29 ist der elastische Verteiler 32 befestigt. Im Bereich des Behälters 23 ist die Rastung 42 zu erkennen. Über den Griff 46 kann der Behälter 23 in den Hohlraum 41 geschoben werden, wobei die Rastung 42 in die Gegenrastung 44 greift.

An dem Applikator 21 ist im Bereich des Hohlraums 41 ein Profil 50 in Form einer Bienenwabenstruktur vorgesehen. Durch diese Strukturierung kann bei gleicher mechanischer Stabilität Material gespart werden.

Das System des Applikators 21 besteht aus mehreren Einzelkomponenten die weitestgehend im Spritzgussverfahren hergestellt werden können. Es ist besonders vorteilhaft, wenn der Applikator 21 dazu die folgenden Komponenten umfasst: eine Applikatorspitze 30 inklusive einem Schaumstoffstanzling 32 und einer Leitung 28, einen Verdrängerkolben 24 und einen Behälterteil 23 umfassend eine zweiteilige Patrone (Außen- und Innenpatrone), die mit einer Siegelfolie 36 verschlossen ist.

Die Applikatorspitze 30 besteht zum einen aus einem konstruierten Spritzgussteil und zum anderen aus einem speziell geformten Schaumstoffstanzling 32. Die Spitze dient insbesondere der Oberflächenversorgung im Mundraum. Durch den verwendeten Schaumstoffstanzling 32 wird die Oberfläche der Applikatorspitze 30 so weit vergrößert, dass damit die Versorgung der zu behandelnden Oberfläche erleichtert wird. Zusätzlich wird hierdurch ein unkontrolliertes Ausspritzen der Dentalflüssigkeit 22 während der Aktivierung verhindert.

Vorteilhaft ist bei diesem Verfahren insbesondere der Wegfall eines harten Kunststoffkerns, der bei sogenannten "Microbrush"-Applikatoren mit PA-Filamenten beflockt wird. Dadurch wird eine schonendere Versorgung von empfindlichen Zahnoberflächen ermöglicht.

Der Behälter 23 besteht vorliegend aus zwei ineinander gesteckten Einzelbehältern, die formschlüssig miteinander verbunden werden. Die aufzubewahrende Dentalflüssigkeit 22 wird vom inneren Behälterteil aufgenommen, der aus einem handelsüblichen Massenkunststoff (HDPE) oder Varianten aus BAREX, PEBAX oder Surlyn (von DuPont) hergestellt werden kann. Die äußere Patronenhülse wird aus einem COC-Kunststoff gefertigt, um eine zusätzliche Barrierewirkung für die (Aceton-haltige) Dentalflüssigkeit zu erzielen.

### Bezugszeichenliste

- 1, 21: Applikator
- 2, 22: Dentalflüssigkeit
- 3, 23: Behälter
- 4, 24: Verdrängerkolben
- 6: Stange
- 8, 28: Leitung
- 10, 30: Applikatorspitze
- 11, 31: Kanal
- 12, 32: Verteiler
- 14: Kanal
- 16, 36: Folie
- 18: Hohldorn
- 25: Hohlraum
- 29: flexible Leitung
- 38: Dorn
- 40: Dichtung
- 41: Hohlraum
- 42: Rastung
- 44: Gegenrastung
- 46: Griff
- 48: Volumenverdränger
- 50: Profil

## Patentansprüche

1. Einhändig bedienbarer Applikator (21) zum Aufbewahren und Applizieren einer Dentalflüssigkeit (22) umfassend einen Hohlraum (41), einen Behälter (23) beinhaltend die Dentalflüssigkeit (22) und eine Leitung (28, 29) mit der die Dentalflüssigkeit (22) aus dem Behälter (23) zu einer Oberfläche einer Applikatorspitze (30) des Applikators (21) leitbar ist,
- wobei der Behälter (23) gegen die Leitung verschiebbar ist,
- wobei in der Leitung (28, 29) ein Kanal (31) angeordnet ist, der das Innere des Behälters (23) mit der Applikatorspitze (30) und optional einem daran angeordneten Verteiler (32) verbindet,
- wobei der Applikator an dem der Applikatorspitze (30) gegenüberliegenden Ende des Applikators (21) einen Griff (46) aufweist, über den der Behälter (23) in den Hohlraum (41) drückbar ist, wobei der Hohlraum (41) die Seitenwände des Behälters (23) aufnimmt, wenn der Behälter über die Leitung (28, 29) geschoben wird,
- wobei am Eintritt der Leitung (28, 29) in den Behälter (23) eine Dichtung (40) angeordnet ist, die verhindert, dass Dentalflüssigkeit (22) in den Hohlraum (41) eindringt,
- wobei der Behälter (23) aus einer Folie (36) gefertigt ist oder zumindest bereichsweise eine Folie (36) umfasst, die den Behälter (23) fluiddicht abschließt, wobei an dem Ende des Behälters (23), das der Leitung (28, 29) zugewandt ist, der Behälter durch eine Folie (36) abgeschlossen ist,
- wobei ein Dorn (38) im Applikator (21) angeordnet ist, mit dem die Folie (36) durchstechbar ist, wobei der Behälter (23) mit durchstochener Folie (36) geöffnet wird und, wobei der Dorn (38) im Bereich der Leitung (28, 29) und des Behälters (23) angeordnet ist, besonders bevorzugt als Hohldorn (18) ausgebildet ist, der ein erstes Ende der Leitung (28, 29) bildet,
- wobei der Applikator (21) einen Verdrängerkolben (24) zum Austreiben der Dentalflüssigkeit (22) aus dem Behälter (23) umfasst, und der Verdrängerkolben (24) an einem Ende des Behälters (23) im Behälter (23) angeordnet ist und gegen den Behälter (23) verschiebbar ist, und wobei beim Einschieben der Seitenwände des Behälters (23) in den Hohlraum (41) der Verdrängerkolben (24) relativ zur Leitung (28, 29) an der gleichen Stelle bleibt, so dass die Dentalflüssigkeit (22) aus dem Inneren des Behälters (23) heraus durch die Leitung (28, 29) gedrückt wird, und der Behälter (23) zum Dorn (38) so gelagert ist, dass der Vortriebsmechanismus, der zum Austreiben der Dentalflüssigkeit aus dem Applikator verwendet wird, auch dazu verwendet wird, die Folie (36) mit dem Dorn zu durchstoßen oder aufzuschlitzen, wobei die Öffnung des Behälters mit derselben Kraft erfolgt, mit der auch die Dentalflüssigkeit ausgetragen wird, und eine einhändige Bedienbarkeit des Applikators nicht nur beim Austragen der Dentalflüssigkeit, sondern auch beim Öffnen des Behälters, erreicht wird.

2. Applikator (21) nach Anspruch 1, **dadurch gekennzeichnet, dass** der Behälter (23) mit einer Beschichtung beschichtet ist.

3. Applikator (21) nach Anspruch 2, **dadurch gekennzeichnet, dass**
die Beschichtung eine porenfreie Polymerbeschichtung, vorzugsweise eine Parylenebeschichtung ist.

4. Applikator (21) nach einem der vorangehenden Ansprüche, **dadurch gekennzeichnet, dass** die Leitung (28, 29) eine flexible Leitung (29) im Bereich der Applikatorspitze (30) umfasst.

5. Applikator (21) nach einem der vorangehenden Ansprüche, **dadurch gekennzeichnet, dass** der Applikator (21) einen Verteiler (32) zum Verteilen der Dentalflüssigkeit (22) umfasst, insbesondere an einem zweiten Ende der Leitung (28, 29) an der Applikatorspitze (30), wobei der Verteiler (32) die Oberfläche der Applikatorspitze (30) bildet.

6. Applikator (21) nach Anspruch 5, **dadurch gekennzeichnet, dass**
der Verteiler (32) austauschbar und/oder auswechselbar ist, vorzugsweise auf das erste Ende der Leitung (28, 29) aufsteckbar ist.

7. Applikator (21) nach einem der Ansprüche 4 bis 6, **dadurch gekennzeichnet, dass** die flexible Leitung (29) um mindestens 45° biegbar ist, vorzugsweise um mindestens 90° biegbar ist, besonders bevorzugt um mindestens 100°.

8. Applikator (21) nach einem der Ansprüche 4 bis 7, **dadurch gekennzeichnet, dass** die flexible Leitung (29) plastisch deformierbar ist.

9. Applikator (21) nach einem der vorangehenden Ansprüche, **dadurch gekennzeichnet, dass** die Dentalflüssigkeit (22) Wasser, Aceton, Ethanol, Isopropanol, Ethylessigester, MMA und/oder tert.-Butanon beinhaltet.

10. Applikator (21) nach einem der vorangehenden Ansprüche, **dadurch gekennzeichnet, dass** die Folie (36) eine unbeschichtete Metallfolie ist.

11. Applikator (21) nach einem der vorangehenden Ansprüche, **dadurch gekennzeichnet, dass** der Applikator (21) eine Rastung (42) umfasst, mit der der Verdrängerkolben (24) gegen den Behälter (23) arretierbar ist.

12. Applikator (21) nach Anspruch 11, **dadurch gekennzeichnet, dass**
die Rastung (42) eine Vielzahl von Raststufen hat, wobei die Raststufen manuell überwindbar sind, so dass die Dentalflüssigkeit (22) portionsweise aus dem Behälter (23) applizierbar ist.

13. Applikator (21) nach einem der vorangehenden Ansprüche, **dadurch gekennzeichnet, dass** der Behälter (23) doppelwandig ist und eine innere und eine äußere Wand aufweist.

14. Applikator (21) nach Anspruch 13, **dadurch gekennzeichnet, dass** die innere Wand zumindest bereichsweise aus einem hochdichten Kunststoff gefertigt ist.

15. Applikator nach Anspruch 13 oder 14, **dadurch gekennzeichnet, dass** die äußere Wand aus einem Cyclo-Olefin-Copolymer (COC) gefertigt ist.

## Claims

1. Applicator (21) for one-handed operation for storing and applying a dental liquid (22) comprising a cavity (41), a container (23) containing the dental liquid (22) and a conduit (28, 29) by means of which the dental liquid (22) is guidable from the container (23) to a surface of an applicator tip (30) of the applicator (21),
- the container (23) being slideable relative to the conduit,
- a channel (31) being arranged in the conduit (28, 29), connecting the inside of the container (23) to the applicator tip (30) and, optionally, to a distributor (32) arranged thereto,
- the applicator having a handhold (46) at the end of the applicator (21) opposing the applicator tip (30), by means of which the container (23) is pushable into the cavity (41), the cavity (41) taking up the side walls of the container (23) when the container is being slid over the conduit (28, 29),
- a seal (40) being arranged at the ingress of the conduit (28, 29) into the container (23) preventing the dental liquid (22) from ingressing into the cavity (41),
- the container (23) being made from a foil (36) or at least regions thereof comprising a foil (36) closing the container (23) in a fluid-tight manner, the container being closed by a foil (36) at the end of the container (23) facing the conduit (28, 29),
- a mandrel (38) being arranged in the applicator (21) by means of which the foil (36) is puncturable, the container (23) with punctured foil (36) being opened, and the mandrel (38) being arranged in the region of the conduit (28, 29) and of the container (23), particularly preferably being formed as hollow mandrel (18) forming a first end of the conduit (28, 29),
- the applicator (21) comprising a displacing piston (24) for extruding the dental liquid (22) from the container (23), and the displacing piston (24) being arranged at one end of the container (23) inside the container (23) and being slideable relative to the container (23), and the displacing piston (24) remaining in the same place relative to the conduit (28, 29) when the side walls of the container (23) are being slid into the cavity (41) so that the dental liquid (22) is being pushed out of the inside of the container (23) through the conduit (28, 29), and the container (23) being positioned with respect to the mandrel (38) such that the driving mechanism which is used for extruding the dental liquid from the applicator is also used to puncture or slit the foil (36) with the mandrel, the container being opened by the same force by means of which the dental liquid is dispensed, and one-handed operability of the applicator being attained not only when the dental liquid is being dispensed but also when the container is being opened.

2. Applicator (21) according to claim 1, **characterised in that**
the container (23) is coated with a coating.

3. Applicator (21) according to claim 2, **characterised in that**
the coating is a non-porous polymer coating, preferably a parylene coating.

4. Applicator (21) according to any one of the preceding claims, **characterised in that**
the conduit (28, 29) comprises a flexible conduit (28, 29) in the region of the applicator tip (30).

5. Applicator (21) according to any one of the preceding claims, **characterised in that**
the applicator (21) comprises a distributor (32) for distributing the dental liquid (22), in particular at a second end of the conduit (28, 29) at the applicator tip (30), the distributor (32) forming the surface of the applicator tip (30).

6. Applicator (21) according to claim 5, **characterised in that**
the distributor (32) is exchangeable and/or replaceable, preferably is pluggable onto the first end of the conduit (28, 29).

7. Applicator (21) according to any one of the claims 4 to 6, **characterised in that**
the flexible conduit (29) is bendable by at least 45°, preferably bendable by at least 90°, particularly preferably by at least 100°.

8. Applicator (21) according to any one of the claims 4 to 7, **characterised in that**
the flexible conduit (29) is plastically deformable.

9. Applicator (21) according to any one of the preceding claims, **characterised in that**
the dental liquid (22) contains water, acetone, ethanol, isopropanol, ethyl acetate, MMA and/or tert.-butanone.

10. Applicator (21) according to any one of the preceding claims, **characterised in that**
the foil (36) is an uncoated metal foil.

11. Applicator (21) according to any one of the preceding claims, **characterised in that**
the applicator (21) comprises a lock-in mechanism (42) by means of which the displacing piston (24) is lockable relative to the container (23).

12. Applicator (21) according to claim 11, **characterised in that**
the lock-in mechanism (42) has a plurality of lock-in steps, the lock-in steps being manually conquerable so that portions of the dental liquid (22) are applicable from the container.

13. Applicator (21) according to any one of the preceding claims, **characterised in that**
the container (23) is double-walled and has an inner and an outer wall.

14. Applicator (21) according to claim 13, **characterised in that**
the inner wall, at least regions thereof, is made from a high-density plastic material.

15. Applicator according to claims 13 or 14, **characterised in that**
the outer wall is made from a cyclo-olefine copolymer (COC).

## Revendications

1. Applicateur (21) pour opération à une main pour stocker et appliquer un liquide dentaire (22) comprenant une cavité (41), un récipient (23) contenant le liquide dentaire (22) et une conduite (28, 29) au moyen de laquelle le liquide dentaire (22) est guidable du récipient (2) à une surface d'une pointe d'applicateur (30) de l'applicateur (21),
- le récipient (23) étant coulissable par rapport à la conduite,
- un canal (31) étant disposé dans la conduite (28, 29), connectant l'intérieur du récipient (23) à la pointe d'applicateur (30) et, facultativement, à un distributeur (32) disposé à celui-ci,
- l'applicateur ayant une poignée (46) à l'extrémité de l'applicateur (21) opposant la pointe d'applicateur (30), au moyen de laquelle le récipient (23) est poussable dans la cavité (41), la cavité (41) accueillant les parois latérales du récipient (23) lorsque le récipient est coulissé sur la conduite (28, 29),
- un joint (40) étant disposé à l'entrée de la conduite (28, 29) dans le récipient (23) empêchant le liquide dentaire (22) de pénétrer dans la cavité (41),
- le récipient (23) étant fabriqué en un film (36) ou, au moins par régions, comprenant un film (36) fermant le récipient (23) de manière étanche au fluide, le récipient étant fermé par un film (36) à l'extrémité du récipient (23) faisant face à la conduite (28, 29),
- un mandrin (38) étant disposé dans l'applicateur (21) au moyen duquel le film (36) est perçable, le récipient (23) avec le film percé (36) étant ouvré, et le mandrin (38) étant disposé dans la région de la conduite (28, 29) et du récipient (23), de préférence particulière étant formé comme mandrin creux (18) formant une première extrémité de la conduite (28, 29),
- l'applicateur (21) comprenant un piston de déplacement (24) pour extruder le liquide dentaire (22) du récipient (23), et le piston de déplacement (24) étant disposé à une extrémité du récipient (23) dans le récipient (23) et étant coulissable par rapport au récipient (23), et le piston de déplacement (24) restant dans la même position par rapport à la conduite (28, 29) lorsque les parois latérales du récipient (23) sont coulissées dans la cavité (41) de sorte que le liquide dentaire (22) est poussé de l'intérieure du récipient (23) par la conduite (28, 29), et le récipient étant positionné quant au mandrin (38) de sorte que le mécanisme de propulsion qui est utilisé pour extruder le liquide dentaire de l'applicateur est aussi utilisé pour percer ou entailler le film (36) avec le mandrin, le récipient étant ouvré par la même force au moyen de laquelle le liquide dentaire est dispensé, et une opérabilité à une main de l'applicateur est atteint non seulement lors du déchargement du liquide dentaire mais aussi lors de l'ouverture du récipient.

2. Applicateur (21) selon la revendication 1, **caractérisé en ce que**
le récipient (23) est revêtu d'un revêtement.

3. Applicateur (21) selon la revendication 2, **caractérisé en ce que**
le revêtement est un revêtement polymère non poreux, de préférence un revêtement parylène.

4. Applicateur (21) selon l'une des revendications précédentes, **caractérisé en ce que**
la conduite (28, 29) comprend une conduite flexible (28, 29) dans la région de la pointe d'applicateur (30).

5. Applicateur (21) selon l'une des revendications précédentes, **caractérisé en ce que**
l'applicateur (21) comprend un distributeur (32) pour distribuer le liquide dentaire (22), en particulière à une deuxième extrémité de la conduite (28, 29) à la pointe d'applicateur (30), le distributeur (32) formant la surface de la pointe d'applicateur (30).

6. Applicateur (21) selon la revendication 5, **caractérisé en ce que**
le distributeur (32) est échangeable et/ou remplaçable, de préférence est enfichable dans la première extrémité de la conduite (28, 29).

7. Applicateur (21) selon l'une des revendications 4 à 6, **caractérisé en ce que**
la conduite flexible (29) est pliable d'au moins 45°, de préférence pliable d'au moins 90°, de préférence particulière d'au moins 100°.

8. Applicateur (21) selon l'une des revendications 4 à 7, **caractérisé en ce que**
la conduite flexible (29) est plastiquement déformable.

9. Applicateur (21) selon l'une des revendications précédentes, **caractérisé en ce que**
le liquide dentaire (22) contient l'eau, l'acétone, l'éthanol, l'isopropanol, l'acétate d'éthyle, le MMA et/ou le tert.-butanone.

10. Applicateur (21) selon l'une des revendications précédentes, **caractérisé en ce que**
le film (36) est un film métallique non revêtu.

11. Applicateur (21) selon l'une des revendications précédentes, **caractérisé en ce que**
l'applicateur (21) comprend un mécanisme de verrouillage (42) au moyen duquel le piston de déplacement (24) est verrouillable par rapport au récipient (23).

12. Applicateur (21) selon la revendication 11, **caractérisé en ce que**
le mécanisme de verrouillage (42) a une pluralité des étapes de verrouillage, les étapes de verrouillage étant manuellement surmontables de sorte que des portions de la liquide dentaire (22) sont applicables du récipient.

13. Applicateur (21) selon l'une des revendications précédentes, **caractérisé en ce que**
le récipient (23) est à double paroi et a une paroi intérieure et extérieure.

14. Applicateur (21) selon la revendication 13, **caractérisé en ce que**
la paroi intérieure, au moins par régions, est fabriquée en une matière plastique de haute densité.

15. Applicateur selon les revendications 13 ou 14, **caractérisé en ce que**
la paroi extérieure est fabriquée en un copolymère cyclo-oléfine (COC).
